# EUROPEAN PATENT SPECIFICATION

(11) **EP 3 210 599 B1**
(45) Date of publication and mention of the grant of the patent: **28.08.2024**
(21) Application number: 17000145.7
(22) Date of filing: 30.01.2017
(51) Int. Cl.: A61K 9/16, A61K 9/20, A61K 31/496

(54) **GASTRO-RESISTANT FORMULATION CONTAINING POSACONAZOLE AND A POLYMERIC PRECIPITATION INHIBITOR**
MAGENRESISTENTE FORMULIERUNG MIT POSACONAZOL UND POLYMERFÄLLUNGSINHIBITOR
FORMULATION GASTRO-RÉSISTANTE CONTENANT DU POSACONAZOLE ET UN INHIBITEUR DE PRÉCIPITATION POLYMÈRE

(30) Priority: 26.02.2016 IN 201611006794
(43) Date of publication of application: 30.08.2017
(60) Divisional application: 21182104.6
(73) Proprietor: Alfred E. Tiefenbacher (GmbH & Co. KG), 22767 Hamburg (DE)
(72) Inventor: Prathap, Vamshi Ramana, 505 452 Telangana (IN); Kalamata, Venkatasimhadri Naidu, Hyderabad 500 072 Telangana (IN); Rallabandi, Bala Ramesha Chary, Hyderabad 500 090 Telangana (IN); Schlehahn, Hendrik, 23843 Travenbrueck (DE); Fitzner, Ansgar, 20253 Hamburg (DE)
(74) Representative: Hamm&Wittkopp Patentanwälte PartmbB

(56) References cited:
- WO-A1-2015/154718
- WO-A1-2017/025292
- WO-A2-2009/129300
- WO-A2-2014/081581
- CN-A- 104 546 724
- US-A1- 2015 231 081

## Description

The present invention is defined in the appended claims and relates to a gastro-resistant pharmaceutical composition comprising posaconazole and a polymeric precipitation inhibitor.

Posaconazole is a triazole antifungal drug marketed under the tradename Noxafil^{®} as a solution for injection, oral suspension and gastro-resistant tablet for the treatment and prophylaxis of invasive fungal infections. Noxafil^{®} is in particular indicated for the prophylaxis of invasive *Aspergillus* and *Candida* infections in severely immuno-compromised patients, such as hematopoietic stem cell transplant recipients with a graft-versus-host-disease and patients with hematologic malignancies with prolonged neutropenia from chemotherapy. The oral suspension is indicated for the treatment of oropharyngeal candidiasis.

Posaconazole is a highly lipophilic weak base exhibiting low aqueous solubility.

Posaconazole's bioavailability in the oral suspension is significantly enhanced when coadministered with food. For this reason, the oral suspension should be administered during or immediately following a full meal to enhance the oral absorption of the drug. The gastro-resistant tablet has an improved bioavailability and can be administered without regard to food.

It is commonly known that the dissolution behavior of a drug depends on its solid state. Different crystalline forms of a drug usually exhibit different dissolution profiles, whereby amorphous forms are generally much more soluble than their crystalline counterparts. In addition, the chemical and physical stability of a drug are dependent on the solid state. Quite often, metastable crystalline or amorphous forms of a drug have to be stabilized in the pharmaceutical composition in order to prevent chemical degradation and interconversion of the crystalline forms/recrystallization of the amorphous form and, thus, fluctuations in the bioavailability.

WO 99/18097 discloses the crystalline forms I, II and III of posaconazole. Form I is the most stable form that does not convert into any other crystalline form under normal storage conditions or under specific stress conditions. The crystalline forms II and III convert into the form I at temperatures between 100 and 125°C.

WO 2009/147075 discloses the crystalline form Y of posaconazole. The form Y is as stable as form I but has a better water solubility, which results in an improved bioavailability.

WO 2010/000668 reports that the crystalline form IV of posaconazole has a better stability in an aqueous suspension and a better water solubility as form I due to a smaller particle size and, thus, larger specific surface area. The crystalline form IV can be directly used for a pharmaceutical composition, i.e. without the need of reducing the particle size by micronization.

WO 2011/158248 discloses the crystalline form V of posaconazole, while WO 2011/003992 discloses the crystalline form II-S from which the other crystalline forms, in particular the crystalline form IV may be obtained.

As an alternative approach for overcoming the solubility problems encountered with posaconazole, WO 98/00113 suggests a pharmaceutical composition comprising a solid solution of the drug within a polymer. The solid solution is prepared by dissolving the drug and a soluble polymer in a suitable organic solvent, followed by removing the solvent, or by dissolving the drug in a suitable organic solvent and adding an insoluble polymer, followed by absorbing the solution into the insoluble polymeric matrix. Preferably, the polymer is povidone or crospovidone.

WO 2009/129301 discloses a solid solution of posaconazole within hydroxypropyl methylcellulose acetate succinate (HPMCAS) by spray-drying a solution containing the drug and the polymer. It is further suggested that the solid solutions may be prepared by using hot-melt extrusion.

WO 2009/129300 discloses the preparation of a solid solution containing posaconazole within a hydroxypropyl methylcellulose derivative, preferably HPMCAS. It has been found that posaconazole forms a solution with the polymer behaving as a eutectic having a melting point below the melting point of the drug (about 169°C). Hence, the use of hydroxypropyl methylcellulose derivatives for the preparation of the solid solution minimizes thermal decomposition and oxidation of posaconazole during the preparation compared to processes which utilize higher melting polymers.

US 2015/0231081 describes the preparation of a solid solution containing posaconazole within a polymer other than hydroxypropyl methylcellulose derived polymers by hot-melt extrusion. Polyvinylpyrrolidone and poly(methacrylic acid/ethyl acrylate) (Eudragit^{®} L 100-55) are disclosed as suitable polymers for the preparation of the solid solution.

WO 2015/154718 discloses the preparation of solid solutions of posaconazole within poly(vinylpyrrolidone/vinylacetate) (copovidone) or polyvinylcaprolactam/polyvinylacetate/polyethylene glycol graft copolymer (e.g. Soluplus^{®}) by hot-melt extrusion.

Hence, the prior art teaches that the aqueous solubility of posaconazole may be enhanced by forming a solid solution of posaconazole within a polymer, i.e. by molecularly dispersing the drug in a suitable polymer. Posaconazole's bioavailability may be further improved if an enteric polymer is used for the preparation of the solid solution because any food effect is minimized when using gastro-resistant formulations; alternatively, posaconazole may be dissolved within a non-enteric polymer if the resulting solid solution is covered by an enteric coating.

In view of the above described state of the art, the objective underlying the present invention was the provision of a gastro-resistant pharmaceutical composition exhibiting enhanced bioavailability of posaconazole. This objective is attained by the subject matter as defined in the claims.

The pharmaceutical composition of the present invention is a gastro-resistant pharmaceutical composition. Gastro-resistant formulations are designed to release the drug in the intestines. According to the European Pharmacopoeia 8.0, gastro-resistant dosage forms are delayed-release dosage forms that are intended to resist the gastric fluid and to release their drug(s) in the intestinal fluid. Gastro-resistance minimizes the food effect of the pharmaceutical composition of the present invention and, thus, improves the bioavailability of the drug. It has been found that the drug's bioavailability is further enhanced if the composition contains a water soluble neutral or anionic polysaccharide. Generally, water soluble means a solubility of ≥ 0.03 g of the solute in 1 ml water at 20 °C. Hence, the gastro-resistant pharmaceutical composition of the present invention comprises posaconazole, an enteric polymer and a water soluble neutral or anionic polysaccharide, wherein posaconazole is molecularly dispersed in the enteric polymer.

It has been found that water soluble neutral or anionic polysaccharides inhibit recrystallization of posaconazole in the intestinal fluid. They act, therefore, as crystallization or precipitation inhibitors. Precipitation inhibitors are compounds capable to stabilize the supersaturation stage of the drug, i.e. they are able to prevent nucleation of the drug molecules or the growing of the initially formed drug particles, which is achieved by covering the surface of the drug particles, thereby preventing particle-particle interaction, or by enhancing the viscosity of the suspension medium. The ability of precipitation inhibitors to kinetically stabilize the supersaturated state of the drug is thought to result from intermolecular interactions between the drug and polymer in solution (e.g. via hydrogen bonding or hydrophobic interactions), the ability of the polymer to sterically hinder the crystallization process or from increasing the viscosity of the suspension medium, and not by enhancing the solubility of the drug, i.e. by increasing the equilibrium solubility (Journal of Drug Targeting 2010, 18(10), 704-731; Adv. Polym. Sci. 1993, 107, 199-265, 244). Hence, neutral or anionic polysaccharides, which are suitable for the composition of the present invention, are typically those used as suspending or thickening agents in pharmaceutical formulations.

The water soluble neutral or anionic polysaccharide is selected from a water soluble cellulose ether, starch and a starch derivative. Examples of a suitable water soluble cellulose ether include hydroxyalkyl cellulose, methylcellulose, methyl ethylcellulose, sodium carboxymethylcellulose (NaCMC) and carboxymethyl ethylcellulose (CMEC). Preferably, the hydroxyalkyl cellulose is selected from hydroxypropyl methylcellulose (HPMC or hypromellose), hydroxyethyl methylcellulose (HEMC or hymetellose), hydroxypropylcellulose (HPC or hyprolose), hydroxyethylcellulose (HEC), hydroxymethylcellulose (HMC), hydroxypropyl ethylcellulose (HPEC), ethyl hydroxyethylcellulose (EHEC) and sodium carboxymethyl hydroxyethylcellulose (NaCMHEC). Examples of suitable starchs include maize (corn) starch, pea starch, potato starch, rice starch, tapioca starch, and wheat starch; and examples of suitable starch derivatives include pregelatinized starch and hydroxypropyl starch.

The enteric polymer is selected from hypromellose derivatives, cellulose derivatives, polyvinylacetate derivatives and polymethacrylic acid derivatives. Examples of hypromellose derivatives include hydroxypropyl methylcellulose phthalate (HPMCP, e.g. available as HP-50 or HP-55 from Shin-Etsu Chemical Co., Ltd. Japan), hydroxypropyl methylcellulose succinate and hydroxypropyl methylcellulose acetate succinate (HPMCAS, e.g. available as AQOAT^{®} from Shin-Etsu Chemical Co., Ltd. Japan). An example of a polyvinylacetate derivative is polyvinylacetate phthalate (PVAP), while examples of cellulose derivatives include cellulose acetate phthalate (CAP), cellulose acetate terephthalate, cellulose acetate isophthalate, cellulose acetate butyrate (CAB), cellulose acetate trimellitate (CAT), methylcellulose acetate phthalate and hydroxypropylcellulose acetate phthalate. Examples of polymethacrylic acid derivatives include poly(methacrylic acid/methyl methacrylate) 1:1 (e.g. available as Eudragit^{®} L 100 from Evonik, Germany), poly(methacrylic acid/methyl methacrylate) 1:2 (e.g. available as Eudragit^{®} S 100 from Evonik, Germany) and poly(methacrylic acid/ethyl acrylate) (e.g. available as Kollicoat^{®} MAE from BASF SE, Germany). According to a preferred embodiment of the present invention, the enteric polymer is a polymethacrylic acid derivative selected from poly(methacrylic acid/methyl methacrylate) and poly(methacrylic acid/ethyl acrylate).

The gastro-resistant pharmaceutical composition of the present invention is prepared by subjecting a mixture containing posaconazole and the enteric polymer to spray-drying or hot-melt extrusion, whereby hot-melt extrusion is preferred. The mixture to be subjected to spray-drying or hot-melt extrusion may additionally contain a non-enteric polymer other than a neutral or anionic polysaccharide that is preferably selected from polyvinylpyrrolidone (povidone), poly(vinylpyrrolidone/vinylacetate) (copovidone), polyvinylcaprolactam/ polyvinylacetate/polyethylene glycol graft copolymer, polyethylene glycol/polyvinyl alcohol graft copolymer, polyethylene oxide, polypropylene oxide, copolymers of ethylene oxide and propylene oxide, polyacrylates, polymethacrylates, vinylacetate polymers such as copolymers of vinyl acetate and crotonic acid, polyvinylalcohol, partially saponified polyvinylalcohol, macrogolglycerol hydroxystearate and polyethylene glycol.

The pharmaceutical composition of the present invention may contain an antioxidant. Preferably, the antioxidant is contained in the mixture comprising posaconazole and the enteric polymer. Examples of antioxidants include butylated hydroxytoluene (BHT), butylated hydroxyanisole (BHA), sodium metabisulfite, ascorbic acid, tocopheryl polyethylene glycol succinate (TPGS) and propyl gallate. Preferably, the antioxidant is propyl gallate.

The mixture contained in the composition of the present invention may additionally contain a monomeric plasticizer, e.g. triethyl citrate, triacetin, dibutyl sebacate, diethyl phthalate, glycerylmonostearate, glycerin and propylene glycol.

Posaconazole has a melting point of 170-172°C, but it degrades at temperatures above 160°C. Hence, the hot-melt extrusion used for the preparation of the gastro-resistant pharmaceutical composition of the present invention has to be conducted at temperatures below 160°C. Preferably, the hot-melt extrusion is conducted at a temperature of 40-160°C, more preferred at a temperature of 120-150°C. The hot-melt extrusion has to be carried out at a temperature that allows the dissolution of the posaconazole used as starting material within the enteric polymer. In principle, any crystalline form of posaconazole as well as the amorphous form may be used for the preparation of the gastro-resistant pharmaceutical composition of the present invention.

The temperature of the hot-melt extrusion can be decreased when using a mixture of the enteric polymer and the non-enteric polymer other than a neutral or anionic polysaccharide, so that it is possible to process polymers with relatively high glass transition temperatures. In addition, since the hot-melt extrusion works at relatively low temperatures, it is possible to use relatively volatile antioxidants, e.g. BHA and BHT, as well as antioxidants, which degrade at processing temperatures above 140°C, e.g. sodium metabisulfite.

Typically, the extrudate contains the enteric polymer and a non-enteric polymer other than a neutral or anionic polysaccharide in a weight ratio of 6:1 to 1:1, preferably of 4:1 to 2:1 and more preferred of 3:1 to 2.5:1. Furthermore, the weight ratio of posaconazole to total polymer content of the extrudate is from 1:1 to 1:5, preferably from 1:2 to 1:4, and more preferred 1:2 to 1:3. According to a preferred embodiment of the present invention the enteric polymer is poly(methacrylic acid/ethyl acrylate) and the non-enteric polymer is selected from poly(vinylpyrrolidone/vinylacetate), polyethylene glycol, and polyvinylpyrrolidone.

It has been found that the presence of a sugar alcohol in the mixture that is subjected to hot-melt extrusion may increase the chemical stability of posaconazole, in particular, if an acidic polymer as poly(methacrylic acid/ethyl acrylate) is present. Preferred sugar alcohols are xylitol, sorbitol, mannitol, maltitol, isomalt, lactitol and erythritol.

The composition according to the present invention preferably contains a relatively high amount of the neutral or anionic polysaccharide crystallization inhibitor. The weight ratio of posaconazole to the neutral or anionic polysaccharide is preferably 3 : 1 to 1 : 2, more preferred 2 : 1 to 1 : 1.5 and most preferred 1.5 : 1 to 1 : 1.4.

The pharmaceutical composition of the present invention is preferably a tablet or a powder to be filled into a capsule. According to a preferred embodiment, the tablet or powder is prepared from granules containing posaconazole molecularly dispersed in the enteric polymer. The granules may be coated with an enteric polymer. It is preferred that the enteric coating of the granules and the enteric polymer constituent of the granules comprise the same enteric polymer. According to a preferred embodiment, the granules consist of posaconazole, an enteric polymer, a non-enteric polymer other than a neutral or anionic polysaccharide, and, optionally, a neutral or anionic polysaccharide, an antioxidant, a sugar alcohol and/or a monomeric plasticizer. It has been found that the neutral or anionic polysaccharide may be used as intragranular or extragranular component in order to provide a sufficiently high precipitation inhibitory effect. Preferably the neutral or anionic polysaccharide is used as extragranular component.

The tablet, which is prepared by compressing the optionally enteric-coated granules of the present invention, may be coated with an enteric polymer or an immediate-release coating, too.

The capsule or tablet of the present invention may contain additional pharmaceutical excipients as extragranular component, e.g. diluents, binders, disintegrants, glidants and lubricants. Examples of diluents include microcrystalline cellulose, calcium hydrogen phosphate, lactose (anhydrous or monohydrate), and calcium carbonate. As binders may be used povidone and copovidone. Examples of disintegrants include croscarmellose sodium, sodium starch glycolate, polyvinylpolypyrrolidone (crospovidone) and low-substituted hydroxypropyl cellulose (L-HPC). As glidants silicone dioxide, talk and the like may be used, while magnesium stearate, calcium stearate, stearic acid, sodium stearyl fumarate and glycerol dibehenate are examples of suitable lubricants.

The following examples are intended to further illustrate the present invention.

### Examples

Hot melt extrusion was performed with a Pharma 11 Twin-screw hot melt extruder from Thermo Fisher Scientific Inc. The used film coating system Opadry II^{®} 85F520152 yellow comprises polyvinyl alcohol, titanium dioxide, polyethylene glycol/macrogol, talc and yellow iron oxide.

The dissolution tests were performed according to general chapter '<711> Dissolution' of the United States Pharmacopeia and the National Formulary (USP38-NF33) and according to the FDA recommended dissolution method for posaconazole delayed-release tablets (dated 06/25/2015, available on the FDA website). The following conditions were used: USP apparatus II (paddle); speed: 75 rpm; acid stage: 0.01 N HCl (750 ml) for 2 h, followed by buffer stage: pH 6.8 phosphate buffer with 0.37% polysorbate 80 (1000 ml); time points: acid stage: 120 min, and buffer stage: 5, 10, 15, 20, 30, 45 and 60 min; samples: 6 units.

### Comparative example and examples 1 to 4

| **Ingredients** | **Comp. example [mg]** | **Ex. 1 [mg]** | **Ex. 2 [mg]** | **Ex. 3 [mg]** | **Ex. 4 [mg]** |
|---|---|---|---|---|---|
| **Stage-A: (Hot-melt extrusion)** | | | | | |
| Posaconazole (Form I) | 100 | 100 | 100 | 100 | 100 |
| Methacrylic acid/ethyl acrylate copolymer (1: 1), Type B (Kollicoat^{®} MAE 100P) | 162 | 162 | 162 | 162 | 162 |
| Triethyl Citrate | 30 | 30 | 30 | 30 | 30 |
| Copovidone (Kollidon^{®} VA 64) | 50 | 50 | 50 | 50 | - |
| Xylitol (Xylisorb^{®} 90) | 20 | 20 | 20 | 20 | 40 |
| Propyl gallate | 2 | 2 | 2 | 2 | 2 |
| Hydroxypropyl cellulose (Klucel EXF Pharma) | - | - | - | - | 75 |
| ***Total weight after HME*** | **364** | **364** | **364** | **364** | **409** |

| **Stage-B: (Blending)** | | | | | |
|---|---|---|---|---|---|
| Posaconazole HME granules | 364 | 364 | 364 | 364 | 409 |
| Hydroxypropyl cellulose (Klucel EXF Pharma) | - | 75 | - | - | - |
| Hydroxyethyl cellulose (Natrosol^{®} 250 LR) | - | - | 75 | - | - |
| HPMC E50 (Methocel E50 Premium LV) | | | - | 75 | - |
| Microcrystalline cellulose (Comprecel^{®} M 102D+) | 111 | 111 | 111 | 111 | 66 |
| Colloidal silicon dioxide (Aerosil^{®} 200 pharma) | 4 | 4 | 4 | 4 | 4 |
| Croscarmellose sodium | 42 | 42 | 42 | 42 | 42 |

| **Stage-C: (Lubrication)** | | | | | |
|---|---|---|---|---|---|
| Sodium stearyl fumarate (Pruv^{®}) | 4 | 4 | 4 | 4 | 4 |
| ***Core tablet weight*** | **525** | **600** | **600** | **600** | **525** |

| **Stage-D: (Film Coating)** | | | | | |
|---|---|---|---|---|---|
| Opadry^{®} II 85F20152 yellow | 18 | 18 | 18 | 18 | 18 |
| Water, purified | q.s. | q.s. | q.s. | q.s. | q.s. |
| ***Coated tablet weight*** | **543** | **618** | **618** | **618** | **543** |

### Process:

Posaconazole and the excipients of stage A were sifted and blended. The mixture was subjected to hot melt extrusion and the obtained extrudate was milled. The excipients of stage B were sifted and blended with the extrudate. The mixture was lubricated with sodium stearyl fumarate and then subjected to compression to obtain a tablet, which was finally film-coated.

**Table 1 Dissolution Tests**

| | **Dissolution [%] in 0.01 N HCl for 2 h followed by pH 6.8 phosphate buffer** | | | | |
|---|---|---|---|---|---|
| **Time (min)** | **Comp. Ex.** | **Ex. 1** | **Ex. 2** | **Ex. 3** | **Ex. 4** |
| **0.01N HCl** | | | | | |
| 120 | 4 | 4 | 4 | 7 | 9 |

| **Phosphate buffer pH 6.8** | | | | | |
|---|---|---|---|---|---|
| 5 | 93 | 94 | 81 | 79 | 88 |
| 10 | 95 | 98 | 94 | 89 | 96 |
| 15 | 95 | 98 | 95 | 89 | 98 |
| 20 | 92 | 98 | 92 | 91 | 99 |
| 30 | 80 | 97 | 89 | 90 | 98 |
| 45 | 58 | 96 | 75 | 88 | 95 |
| 60 | 40 | 95 | 54 | 88 | 95 |

### Examples 5 to 8

| **Ingredients** | **Ex. 5 [mg]** | **Ex. 6 [mg]** | **Ex. 7 [mg]** | **Ex. 8 [mg]** |
|---|---|---|---|---|
| **Stage-A: (Hot-melt extrusion)** | | | | |
| Posaconazole (Form I) | 100 | 100 | 100 | 100 |
| Methacrylic acid/ethyl acrylate copolymer (1:1), Type B (Kollicoat^{®} MAE 100P) | 162 | 162 | 162 | 162 |
| Triethyl Citrate | 30 | 30 | 30 | 30 |
| Copovidone (Kollidon^{®} VA 64) | 50 | 50 | 50 | 50 |
| Xylitol (Xylisorb^{®} 90) | 20 | 20 | 20 | 20 |
| Propyl gallate | 2 | 2 | 2 | 2 |
| ***Total weight after HME*** | **364** | **364** | **364** | **364** |

| **Stage-B: (Blending)** | | | | |
|---|---|---|---|---|
| Posaconazole HME granules | 364 | 364 | 364 | 364 |
| Pregelatinized starch (Starch 1500) | 75 | - | - | - |
| Hydroxypropyl starch (Lycoat RS 720) | - | 75 | - | - |
| HPMC E50 (Methocel E50 Premium LV) | - | - | 100 | 37.5 |
| Hydroxyethyl cellulose (Natrosol^{®} 250 LR) | - | - | - | 37.5 |
| Microcrystalline cellulose (Comprecel^{®} M 102D+) | 111 | 111 | 86 | 111 |
| Colloidal silicon dioxide (Aerosil^{®} 200 pharma) | 4 | 4 | 4 | 4 |
| Croscarmellose sodium | 42 | 42 | 42 | 42 |

| **Stage-C: (Lubrication)** | | | | |
|---|---|---|---|---|
| Sodium stearyl fumarate (Pruv^{®}) | 4 | 4 | 4 | 4 |
| ***Core tablet weight*** | **600** | **600** | **600** | **600** |

| **Stage-D: (Film Coating)** | | | | |
|---|---|---|---|---|
| Opadry^{®} II 85F20152 yellow | 18 | 18 | 18 | 18 |
| Water, purified | q.s. | q.s. | q.s. | q.s. |
| ***Coated tablet weight*** | **618** | **618** | **618** | **618** |

### Process:

Posaconazole and the excipients of stage A were sifted and blended. The mixture was subjected to hot melt extrusion and the obtained extrudate was milled. The excipients of stage B were sifted and blended with the extrudate. The mixture was lubricated with sodium stearyl fumarate and then subjected to compression to obtain a tablet, which was finally film-coated.

**Table 2 Dissolution Tests**

| | **Dissolution [%] in 0.01 N HCl for 2 h followed by pH 6.8 phosphate buffer** | | | |
|---|---|---|---|---|
| **Time (min)** | **Ex. 5** | **Ex. 6** | **Ex. 7** | **Ex. 8** |
| **0.01N HCl** | | | | |
| 120 | 4 | 4 | 4 | 4 |

| **Phosphate buffer pH 6.8** | | | | |
|---|---|---|---|---|
| 5 | 94 | 92 | 100 | 101 |
| 10 | 94 | 94 | 102 | 102 |
| 15 | 94 | 94 | 102 | 104 |
| 20 | 94 | 93 | 101 | 103 |
| 30 | 98 | 96 | 102 | 102 |
| 45 | 94 | 92 | 102 | 105 |
| 60 | 78 | 89 | 101 | 102 |

## Claims

1. A gastro-resistant pharmaceutical composition comprising posaconazole, an enteric polymer and a water soluble neutral or anionic polysaccharide, wherein posaconazole is molecularly dispersed in the enteric polymer by hot-melt extrusion, wherein the enteric polymer is selected from hydroxypropyl methylcellulose phthalate, hydroxypropyl methylcellulose succinate, polyvinylacetate phthalate, cellulose acetate phthalate, cellulose acetate terephthalate, cellulose acetate isophthalate, cellulose acetate butyrate, cellulose acetate trimellitate, methylcellulose acetate phthalate and hydroxypropyl cellulose acetate phthalate, poly(methacrylic acid/methyl methacrylate) 1:1, poly(methacrylic acid/methyl methacrylate) 1:2 and poly(methacrylic acid/ethyl acrylate), and wherein the water soluble neutral or anionic polysaccharide is selected from a water soluble cellulose ether, starch and a starch derivative.

2. A gastro-resistant pharmaceutical composition according to claim 1, wherein the cellulose ether is selected from hydroxyalkyl cellulose, methylcellulose, methyl ethylcellulose, sodium carboxymethylcellulose (NaCMC) and carboxymethyl ethylcellulose (CMEC).

3. A gastro-resistant pharmaceutical composition according to claim 2, wherein the hydroxyalkyl cellulose is selected from hydroxypropyl methylcellulose (HPMC or hypromellose), hydroxyethyl methylcellulose (HEMC or hymetellose), hydroxypropylcellulose (HPC or hyprolose), hydroxyethylcellulose (HEC), hydroxymethylcellulose (HMC), hydroxypropyl ethylcellulose (HPEC), ethyl hydroxyethylcellulose (EHEC) and sodium carboxymethyl hydroxyethylcellulose (NaCMHEC).

4. A gastro-resistant pharmaceutical composition according to claim 1, wherein the starch derivative is selected from pregelatinized starch and hydroxypropyl starch.

5. The composition according to any one of the preceding claims, wherein the enteric polymer is selected from poly(methacrylic acid/methyl methacrylate) and poly(methacrylic acid/ethyl acrylate).

6. The composition according to any one of the preceding claims, wherein the weight ratio of posaconazole to the neutral or anionic polysaccharide is 3 : 1 to 1 : 2, preferably 2 : 1 to 1 : 1.5, and more preferred 1.5 : 1 to 1 : 1.4.

7. The composition according to any one of the preceding claims, wherein the composition is a tablet or a powder to be filled into a capsule.

8. The composition according to claim 7, wherein the tablet or the powder is prepared from granules containing posaconazole molecularly dispersed in the enteric polymer.

9. The composition according to claim 8 containing the neutral or anionic polysaccharide as intragranular or extragranular component.

## Patentansprüche

1. Magensaftresistente pharmazeutische Zusammensetzung, umfassend Posaconazol, ein enterisches Polymer und ein wasserlösliches, neutrales oder anionisches Polysaccharid, wobei das Posaconazol in dem enterischen Polymer mittels Schmelzextrusion molekular dispergiert ist, wobei das enterische Polymer ausgewählt ist aus Hydroxypropylmethylcellulosephthalat, Hydroxypropylmethylcellulosesuccinat, Polyvinylacetatphthalat, Celluloseacetatphthalat, Celluloseacetatterephthalat, Celluloseacetatisophthalat, Celluloseacetatbutyrat, Celluloseacetattrimellitat, Methylcelluloseacetatphthalat und Hydroxypropylcelluloseacetatphthalat, Poly(methacrylsäure/methylmethacrylat) 1:1, Poly(methacrylsäure/methylmethacrylat) 1:2 und Poly(methacrylsäure/ethylacrylat), und wobei das wasserlösliche, neutrale oder anionische Polysaccharid aus einem wasserlöslichen Celluloseether, Stärke und einem Stärkederivat ausgewählt ist.

2. Magensaftresistente pharmazeutische Zusammensetzung gemäß Anspruch 1, wobei der Celluloseether aus Hydroxyalkylcellulose, Methylcellulose, Methylethylcellulose, Natriumcarboxymethylcellulose (NaCMC) und Carboxymethylethylcellulose (CMEC) ausgewählt ist.

3. Magensaftresistente pharmazeutische Zusammensetzung gemäß Anspruch 2, wobei die Hydroxyalkylcellulose ausgewählt ist aus Hydroxypropylmethylcellulose (HPMC oder Hypromellose), Hydroxyethylmethylcellulose (HEMC oder Hymetellose), Hydroxypropylcellulose (HPC oder Hyprolose), Hydroxyethylcellulose (HEC), Hydroxymethylcellulose (HMC), Hydroxypropylethylcellulose (HPEC), Ethylhydroxyethylcellulose (EHEC) und Natriumcarboxymethylhydroxyethylcellulose (NaCMHEC).

4. Magensaftresistente pharmazeutische Zusammensetzung gemäß Anspruch 1, wobei das Stärkederivat aus vorverkleisterter Stärke und Hydroxypropylstärke ausgewählt ist.

5. Zusammensetzung gemäß einem der vorstehenden Ansprüche, wobei das enterische Polymer aus Poly(methacrylsäure/methylmethacrylat) und Poly(methacrylsäure/ethylacrylat) ausgewählt ist.

6. Zusammensetzung gemäß einem der vorstehenden Ansprüche, wobei das Gewichtsverhältnis von Posaconazol zu dem neutralen oder anionischen Polysaccharid 3:1 bis 1:2, vorzugsweise 2:1 bis 1:1,5 und mehr bevorzugt 1,5:1 bis 1:1,4 ist.

7. Zusammensetzung gemäß einem der vorstehenden Ansprüche, wobei die Zusammensetzung eine Tablette ist oder ein Pulver, das in eine Kapsel zu füllen ist.

8. Zusammensetzung gemäß Anspruch 7, wobei die Tablette oder das Pulver aus einem Granulat hergestellt ist, das Posaconazol in dem enterischen Polymer molekulardispers enthält.

9. Zusammensetzung gemäß Anspruch 8, die das neutrale oder anionische Polysaccharid als intragranulare oder extragranulare Komponente enthält.

## Revendications

1. Composition pharmaceutique gastro-résistante comprenant du posaconazole, un polymère entérique et un polysaccharide neutre ou anionique soluble dans l'eau, dans laquelle un posaconazole est dispersé de manière moléculaire dans un polymère entérique par extrusion à chaud, dans laquelle un polymère entérique est choisi parmi un phtalate d'hydroxypropylméthylcellulose, un succinate d'hydroxypropylméthyl-cellulose, un phtalate de polyvinylacétate, un phtalate d'acétate de cellulose, un téréphtalate d'acétate de cellulose, un isophtalate d'acétate de cellulose, un butyrate d'acétate de cellulose, un trimellitate d'acétate de cellulose, un phtalate d'acétate de méthylcellulose et un phtalate d'acétate d'hydroxypropylcellulose, un poly(acide méthacrylique/méthacrylate de méthyle) 1:1, un poly(acide méthacrylique/ méthacrylate de méthyle) 1:2 et un poly(acide méthacrylique/acrylate d'éthyle), et dans laquelle un polysaccharide neutre ou anionique soluble dans l'eau est choisi parmi un éther de cellulose soluble dans l'eau, de l'amidon et un dérivé d'amidon.

2. Composition pharmaceutique gastro-résistante selon la revendication 1, dans laquelle l'éther de cellulose est choisi parmi une hydroxyalkylcellulose, une méthylcellulose, une méthyléthylcellulose, une carboxyméthylcellulose sodique (NaCMC) et une carboxyméthyléthylcellulose (CMEC).

3. Composition pharmaceutique gastro-résistante selon la revendication 2, dans laquelle une hydroxyalkylcellulose est choisie parmi une hydroxypropylméthyl-cellulose (HPMC ou hypromellose), une hydroxyéthylméthylcellulose (HEMC ou hymétellose), une hydroxypropylcellulose (HPC ou hyprolose), une hydroxyéthyl-cellulose (HEC), une hydroxyméthylcellulose (HMC), une hydroxypropyléthylcellu-lose (HPEC), une éthylhydroxyéthylcellulose (EHEC) et une carboxyméthylhydroxy-éthylcellulose sodique (NaCMHEC).

4. Composition pharmaceutique gastro-résistante selon la revendication 1, dans laquelle le dérivé d'amidon est choisi parmi un amidon prégélatinisé et un amidon d'hydroxypropyle.

5. Composition selon l'une quelconque des revendications précédentes, dans laquelle le polymère entérique est choisi parmi un poly(acide méthacrylique/ méthacrylate de méthyle) et un poly(acide méthacrylique/acrylate d'éthyle).

6. Composition selon l'une quelconque des revendications précédentes, dans laquelle le rapport pondéral du posaconazole au polysaccharide neutre ou anionique est de 3: 1 à 1: 2, de préférence 2: 1 à 1: 1,5, et plus préférentiellement de 1,5: 1 à 1: 1,4.

7. Composition selon l'une quelconque des revendications précédentes, dans laquelle la composition est un comprimé ou une poudre pour remplir une capsule.

8. Composition selon la revendication 7, dans laquelle le comprimé ou la poudre est préparé(e) à partir de granules contenant du posaconazole dispersé de manière moléculaire dans le polymère entérique.

9. Composition selon la revendication 8, contenant le polysaccharide neutre ou anionique en tant que composant intragranulaire ou extragranulaire.
